# EUROPEAN PATENT APPLICATION

(11) **EP 3 015 541 A1**
(43) Date of publication of application: **04.05.2016**
(21) Application number: 14849470.1
(22) Date of filing: 26.09.2014
(51) Int. Cl.: C12M 1/00

(54) **WORK CHAMBER**

(30) Priority: 30.09.2013 JP 2013203338
(71) Applicant: Panasonic Healthcare Holdings Co., Ltd., Tokyo 105-8433 (JP)
(72) Inventor: KOBAYASHI, Koichi, Ehime 791-0395 (JP); YOKOI, Yasuhiko, Ehime 791-0395 (JP); SEKINE, Hironobu, Ehime 791-0395 (JP)
(74) Representative: Grünecker Patent- und Rechtsanwälte PartG mbB
(86) International application number: PCT/JP2014/075598
(87) International publication number: WO 2015/046413

(57) **Abstract**

A working chamber including: a body case including a work space in an interior thereof; a supply unit configured to supply gas through a supply filter provided at the upper front of the work space; a louver that guides gas to a back surface side of the work space after the gas passes through the supply filter; and an illuminating means positioned lower than the louver and in front of the louver, at least one surface of the louver, which faces the illuminating means, being a reflection surface.

## Description

### [Technical Field]

The present disclosure relates to a working chamber, such as an isolator, a clean bench, or a cabinet, used for a regenerative medical experiment environmental device and a pharmaceutical experiment environmental device.

### [Background]

Patent Literature 1 discloses an isolator that can prevent dust from being mixed into cells and the like and can prevent dust from affecting culture works such as cell manipulation. This isolator supplies gas downward from above a working chamber by an air current control means, and discharges the gas in the working chamber from discharge hole portions provided on a work plate.

### [Citation List]

### [Patent Literature]

[Patent Literature 1] Japanese Patent Application Laid-open Publication No. 2011-177091

### [Summary]

### [Technical Problem]

The present disclosure is to provide a working chamber that improves workability.

### [Solution to Problem]

A working chamber according to the present disclosure includes: a box-shaped body case including a work space in an interior thereof and an insertion portion, in a front surface thereof, to which a worker's arm is inserted; a supply unit configured to supply, into the work space, gas in an exterior of the body case through a supply filter provided at the upper front of the work space in the body case; a louver that guides gas supplied into the work space by the supply unit to a back surface side of the work space after the gas passes through the supply filter; and an illuminating means positioned lower than the louver and in front of the louver in the work space, in an upper part of the work space in the body case, at least one surface of the louver, which faces the illuminating means, being a reflection surface.

### [Advantageous Effects]

A working chamber according to the present disclosure is effective in improving workability.

### [Brief Description of the Drawings]

[Fig. 1]

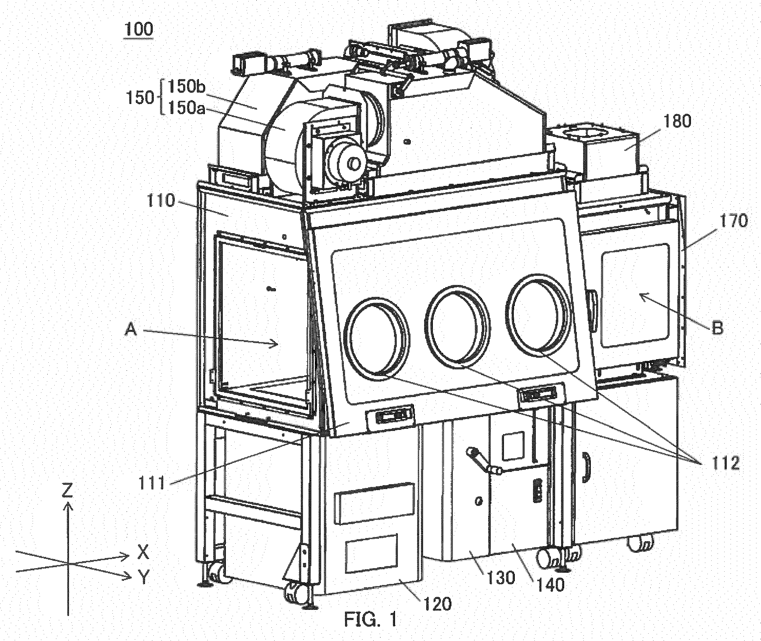

   Fig. 1 is a perspective view illustrating an isolator system according to an embodiment 1.
[Fig. 2]

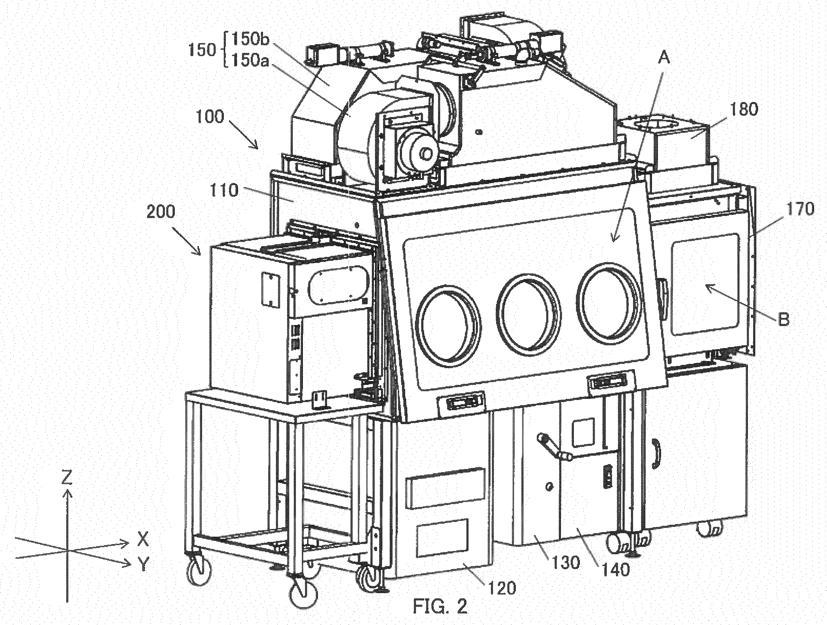

   Fig. 2 is a perspective view illustrating the isolator system when an incubator is mounted thereto according to the embodiment 1.
[Fig. 3]

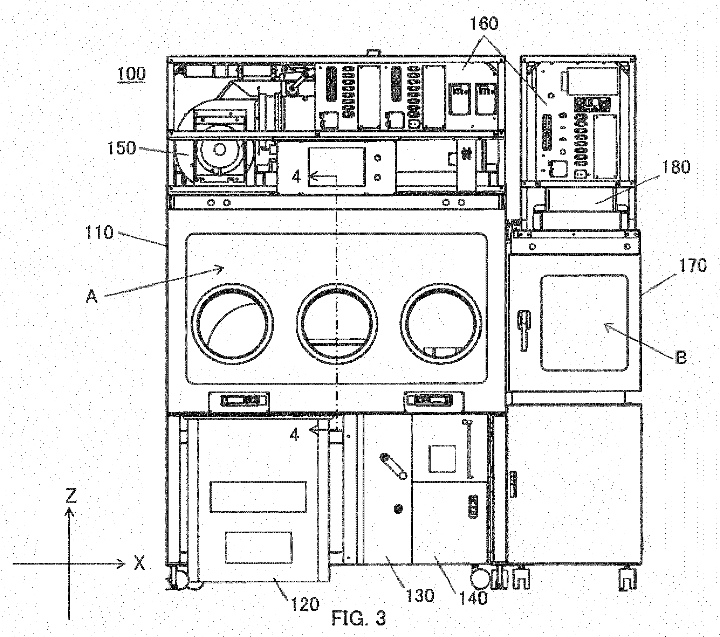

   Fig. 3 is a front view of the isolator system according to the embodiment 1.
[Fig. 4]

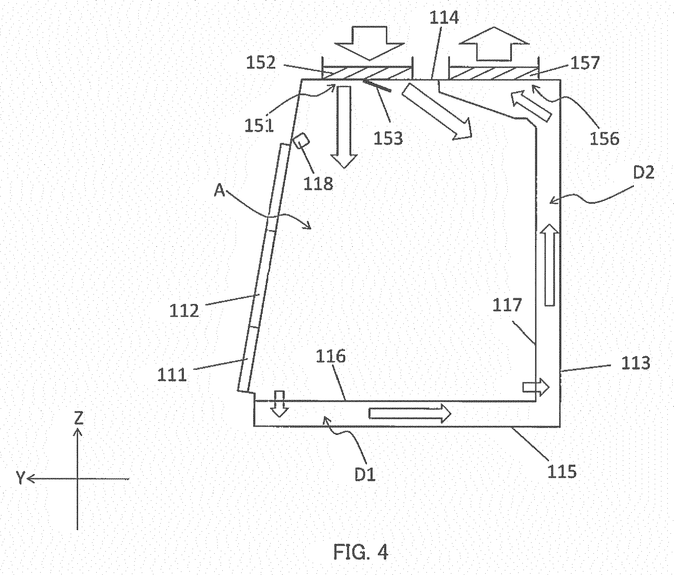

   Fig. 4 is a 4-4 cross-sectional view of a glove box in Fig. 3.
[Fig. 5]

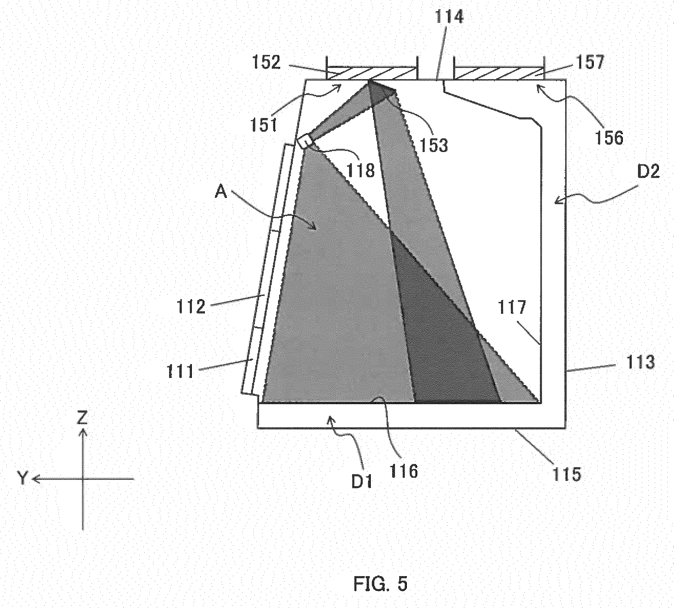

   Fig. 5 is an explanatory view showing a course of an illuminating light from an illuminating unit according to the embodiment 1.
[Fig. 6]

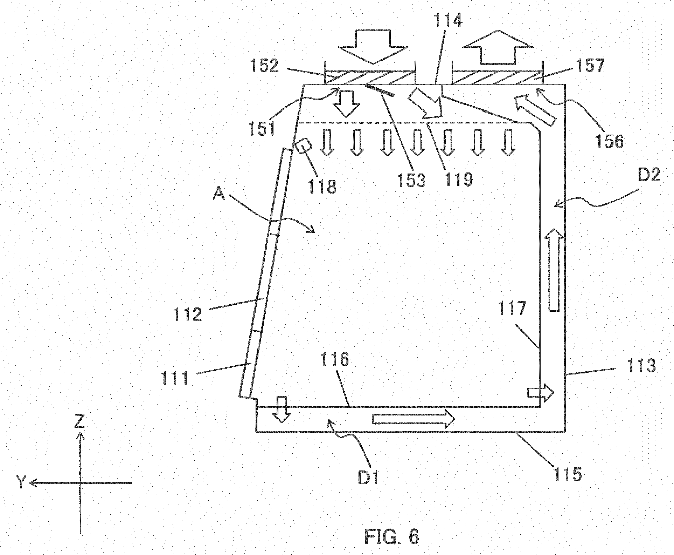

   Fig. 6 is a cross-sectional view of the glove box according to another embodiment.
[Fig. 7]

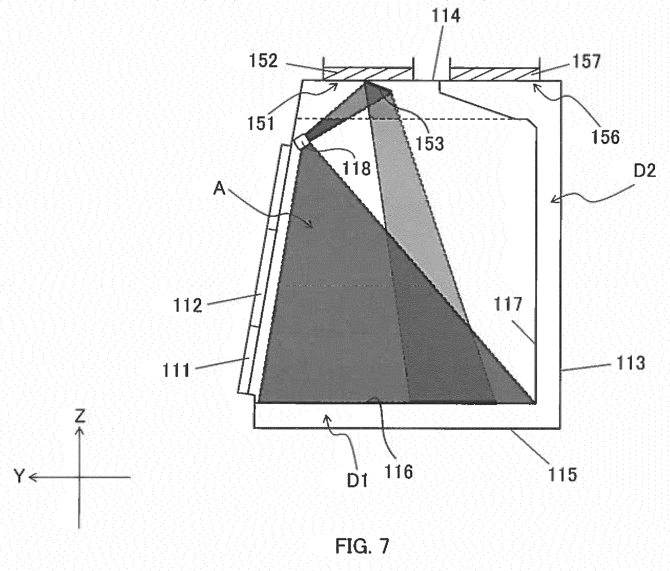

   Fig. 7 is an explanatory view showing a course of an illuminating light from an illuminating unit according to another embodiment.

### [Mode for Carrying Out the Disclosure]

Hereinafter, embodiments will be described in detail with reference to drawings as necessary. However, a description in more detailed than necessary may be omitted. For example, detailed descriptions of well-known matters and the repeated descriptions of substantially the same configurations may be omitted. This is to prevent the following description from being unnecessarily redundant than necessary, and to facilitate the understanding of a person skilled in the art.

Note that the invertors provide the accompanying drawings and the following descriptions to help a person skilled in the art fully understand the present disclosure, and are not intended to limit the subject matters described in the claims thereby.

### (Embodiment 1)

An isolator system 100 will be described hereinafter as an example of a working chamber in an embodiment 1 with reference to Figs. 1 to 5.

The isolator system 100 in the embodiment 1 is a device configured to perform, for example, work for cell culture, manipulation, observation, etc., in a sterilized environment. Note that sterilization means an act of killing microorganisms, cells and the like, to bring a state closer to a sterile environment.

Note that, in the present embodiment, it is assumed that the Z axis is an axis along a vertical direction in which the isolator system 100 is provided in a standing manner, and a direction toward the upper side is +Z direction and a direction toward the lower side (downward) is -Z direction. It is assumed that the Y axis is an axis along a direction perpendicular to the front surface and the back surface of the isolator system 100, and a direction from the front surface, where openings for conducting work in the interior of a work space are provided, toward the back surface opposite to the front surface is -Y direction, and a direction from the back surface toward the front surface is +Y direction. It is assumed that the X axis is an axis along a direction perpendicular to the left and right side surfaces when seen from the front, and a direction from the left side surface toward the right side surface when seen from the front is +X direction, and a direction from the right side surface toward the left side surface is -X direction.

### [1. Isolator System]

An entire configuration of the isolator system 100 will be described with reference to Figs. 1 to 4. Fig. 1 is a perspective view illustrating an isolator system 100 according to an embodiment 1. Fig. 2 is a perspective view illustrating the isolator system 100 when an incubator 200 is mounted thereto according to the embodiment 1. Fig. 3 is a front view of the isolator system 100 according to the embodiment 1. Fig. 4 is a 4-4 cross-sectional view of a glove box 110 in Fig. 3.

As illustrated in Fig. 1, the isolator system 100 the isolator system 100 according to the embodiment 1 includes : a glove box 110; a centrifuge unit 120; an observation unit 130; a sterilization unit 140; an air conditioning unit 150; a control unit 160 (see Fig. 3); a pass box 170; and an air conditioning unit 180.

As illustrated in Fig. 2, in the isolator system 100, the incubator 200 is mounted to the side surface opposite to the side surface to which the pass box 170 is provided in the glove box 110. The incubator 200 includes a storage chamber (not shown) in the interior thereof. The storage chamber is a chamber for storing a culture, and is partitioned as a space to restrain bacterial invasion from the exterior, for example, by a rectangular parallelepiped-shaped box body. The storage chamber is partitioned with, for example, stainless steel plates. The incubator 200 is demountably configured with respect to the isolator system 100. Thus, culture can be controlled in each incubator 200. For example, a dedicated incubator 200 can be used for each donor, thereby restraining occurrence of failures such as mix-up of culture.

As illustrated in Fig. 1, the glove box 110 includes a substantially box-shaped work space A, formed in the interior thereof, that is isolated from the surroundings to conduct work in the sterilized environment. A detailed configuration will be described later. The centrifuge unit 120 is provided below the glove box 110, and can be connected from the work space A. The centrifuge unit 120 includes, in the interior thereof, a centrifuge configured to centrifuge a sample to work with in the work space A. The observation unit 130 is provided below the glove box 110, and can be connected from the work space A. The observation unit 130 includes, in the interior thereof, an observation device configured to observe a sample to work with in the work space A. Further, the observation unit 130 includes: a lifting mechanism capable of lifting and lowering the observation device provided in the interior thereof; and a handle, provided in the exterior, with which the lifting mechanism is operated. A worker operates the lifting mechanism using the handle, so that the observation device can be moved up into the work space A when the observation device is used and the observation device can be housed in the observation unit 130 when the observation unit 130 is not used. The sterilization unit 140 is provided below the glove box 110, to supply a sterilizing substance such as hydrogen peroxide into the glove box 110. The sterilization unit 140 in the present embodiment is configured to spray sterilizing mist, obtained by converting sterilizing liquid into mist, through a nozzle provided in the glove box 110, to sterilize the interior thereof.

The air conditioning unit 150 is provided above the glove box 110, and is configured to control the air conditioning in the interior thereof. The air conditioning unit 150 includes a supply unit 150a and a discharge unit 150b. The air conditioning unit 150 is configured to supply gas into the work space A using the supply unit 150a, and discharge the gas in the work space A using the discharge unit 150b.

As illustrated in Fig. 3, the control unit 160 is provided above the glove box 110 and the pass box 170, and is configured to control the operations of the devices such as the sterilization unit 140 and the air conditioning unit 150.

The pass box 170 is provided to the side surface of the glove box 110 to allow the worker to bring a work object from the exterior into the interior of the work space A. In the interior of the pass box 170, a conveying space B is formed in which the work object is temporarily stored. The conveying space B has airtightness with respect to surrounding environment. Before the work object is inserted into the work space A from the exterior, the work object is sterilized in the conveying space B. An opening for moving the work object is provided to the side surface of the pass box 170 and faces an opening provided to the side surface of the glove box 110, so that the glove box 110 and the pass box 170 are fixed. In this way, the work space A and the conveying space B communicate with each other while maintaining airtightness. The opening of the pass box 170 has an openable/closable door installed thereto. The door can separate the conveying space B from the work space A while maintaining airtightness. The air conditioning unit 180 is provided at the upper portion of the pass box 170, and is configured to control the air conditioning in the conveying space B therein.

### [2. Glove Box]

The configuration of the glove box 110 will be described with reference to Fig. 4. Fig. 4 is a 4-4 cross-sectional view of a glove box in Fig. 3. As illustrated in Fig. 4, in the glove box 110, the box-shaped body case is configured with a front surface plate 111 having a plurality of front surface openings 112, which are insertion portions for worker's hands, a back surface plate 113, a top surface plate 114, a bottom surface plate 115, and left and right side surface plates. The glove box 110 includes, in the interior thereof: a work plate 116 on which work is conducted; and a partitioning plate 117 provided to the back surface side of the glove box. The box shaped work space A that is a space for conducting work is formed with the front surface plate 111, the top surface plate 114, the work plate 116, the partitioning plate 117, and the left and right side surface plates. The glove box 110 is a compartment that is formed with airtightness so as to restrain bacterial invasion from the exterior. In the glove box 110 of the present embodiment, the work plate 116, the partitioning plate 117, the bottom surface plate 115, the back surface plate 113, the top surface plate 114, and the left and right side surface plates are configured with stainless steel plates, which is easily cleaned and sterilized.

Gloves (not shown) are respectively mounted to the plurality of front surface openings 112. The front surface plate 111 is openable/closable about a hinge provided an upper end thereof serving as an axis. Thus, the front surface of the glove box 110 can be opened/closed. Openings for mounting the pass box 170 and the incubator 200 are formed on left and right side surface plates. The pass box 170 is mounted to the opening on the right side surface plate of the glove box 110, and the incubator 200 is mounted to the opening on the left side surface plate thereof. A worker conducts work in the interior of the work space A through gloves at the time of working.

A bottom surface duct D1 is formed between the work plate 116 and the bottom surface plate 115 as a discharge path through which the gas in the work space A is discharged. Further, a back surface duct D2 is formed between the partitioning plate 117 and the back surface plate 113 as a discharge path through which the gas in the work space A is discharged. The bottom surface duct D1 and the back surface duct D2 communicate with each other. The gas supplied from the inlet 151 flows substantially in a direction indicated by arrows in the figure, and passes through the bottom surface duct D1 and the back surface duct D2 to be discharged from the outlet 156. In this way, the gas flow can be stabilized in the work space A and contamination can be suppressed.

The top surface plate 114 in the glove box 110 is provided with an inlet 151 and an outlet 156. The inlet 151 is disposed at the upper front of the work space A, and the outlet 156 is disposed at the upper rear thereof. The gas is supplied into the work space A from the inlet 151, and the gas in the work space A is discharged from the outlet 156. In the glove box 110, a particulate trap filter 152 such as an HEPA filter is mounted to the inlet 151 to secure the sterile environment in the interior, and the gas is supplied through the particulate trap filter 152 into the glove box 110. Further, a particulate trap filter 157 such as the HEPA filter is mounted to the outlet 156, and the gas in the glove box 110 is discharged from the interior of the glove box 110 through the particulate trap filter 157. On the work space A side of the top surface plate 114 in the glove box 110, a louver 153 is provided that guides the gas supplied into the work space A by the supply unit 150a to the back surface side of the work space A, that is, to the partitioning plate 117 side, after the gas passes through the particulate trap filter 152. The upper portion of the louver 153 is fixed to the top surface plate 114 so as to allow the lower portion of the louver 153 to be closer to the back surface side than the upper portion thereof. In other words, the louver 153 is inclined to the back surface side from the upper portion toward the lower portion. Further, the front surface of the louver 153 is mirror-finished to be a reflection surface.

An illuminating unit 118 is provided at the upper front of the work space A in the glove box 110 for easily conducting works by brightly illuminating the interior of the work space A, particularly, the work plate 116. The illuminating unit 118 is positioned lower than the louver 153 and in front of the louver 153 in the work space A. In the isolator system 100, the illuminating unit 118 is provided to the upper front side of the work space A in a horizontal direction.

### [3. Effects, etc.]

Fig. 5 is an explanatory view showing a course of an illuminating light from the illuminating unit 118 according to the embodiment 1.

As stated above, in the present embodiment, the isolator system 100 (working chamber) includes: the box shaped glove box 110 (body case) having the work space A in the interior thereof and the front surface openings 112 (insertion portions) on the front surface thereof, to which a worker' s arms are to be inserted; the supply unit 150a (supply part) configured to supply the gas outside the glove box 110 into the work space A through the particulate trap filter 152 (supply filter) for air supply, which is provided at the upper front of the work space A in the glove box 110; the louver 153 that guides the gas supplied into the work space A by the supply unit 150a to the back surface side of the work space A after the gass passes through the particulate trap filter 152; and the illuminating unit 118 (illuminating means) positioned lower than the louver 153 and in front of the louver 153 in the work space A in the upper portion of the work space A in the glove box 110, and at least one surface of the louver 153, which faces the illuminating unit 118, is a reflection surface.

Thus, not only the gas flow in the work space A is stabilized and contamination is suppressed by allowing the louver 153 to guide the gas supplied from the upper front of the work space A to the back surface side, as illustrated in Fig. 4, but also workability of workers can be improved by reflecting the light of the illuminating unit 118 by the louver 153 to illuminate the work plate 116, as illustrated in Fig. 5.

On the other hand, if a member for collecting the light of the illuminating unit 118 to the work plate 116 is provided separately, the cost is increased due to the increase of the number of parts, and also workability is deteriorated since the work space A becomes narrower.

However, the isolator system 100 in the present embodiment is provided with the louver 153, and thus both suppressing contamination and illuminating the work plate 116 can be configured by one member.

Further, the isolator system 100 includes: the particulate trap filter 157 (discharge filter) for discharging gas, which is provided at the upper rear of the glove box 110; the discharge unit 150b (discharge part) that discharges air in the work space A to the outside through the particulate trap filter 157; and the partitioning plate 117 which is provided on the back surface side of the glove box 110 to form a ventilation passage between the back surface plate 113 of glove box 110 and the partitioning plate 117 and partition air currents into supply air and discharge air.

Consequently, the isolator system 100 can separate the air current supplied into the work space from the air current to be discharged from the work space A. This can further suppress contamination.

### (Other Embodiment)

As described above, the embodiment 1 has been described as an example of the technique disclosed in the present application. However, the present disclosed technique is not limited thereto, but is applicable to an embodiment in which modifications, replacements, additions, omissions and the like are made as appropriate. Further, the components described in the above embodiment 1 can be combined to configure a new embodiment.

Then, other embodiments will hereinafter be exemplified. Note that, the same components as those in the embodiment 1 will be designated by the same reference numerals as those in the embodiment 1, for convenience' sake.

In the embodiment 1, the glove box 110 has been described as an example of the body case. The glove box 110 is not limited to a body case for an isolator in which work is conducted in the work space A through gloves. The present disclosed technique is useful also for a clean bench or a cabinet having the front surface openings 112 to which no glove is mounted. In short, the body case may be the one in which gas is supplied into the work space A to adjust an environment of the work space A, in an experimental environment device in which a worker inserts his/her hand into the work space A to conduct work in the interior thereof.

Further, a top board facing the work plate 116 may be provided at the upper part of the work space A that is formed in the glove box 110 in the embodiment 1. In this case, it is necessary for the top board to be provided with hole portions through which the supplied gas passes. Fig. 6 is a cross-sectional view of a glove box 190 according to another embodiment. The glove box 190 shown in Fig. 6 includes a top board 119 having a plurality of hole portions. Fig. 7 is an explanatory view showing a course of an illuminating light from the illuminating unit 118 according to another embodiment.

As illustrated in Fig. 6, the louver 153 is disposed between the top board 119 and the particulate trap filter 152 for air supply. The illuminating unit 118 is disposed below the top board 119, and the light of the illuminating unit 118 passes through a plurality of hole portions to hit a reflection surface of the louver 153. Then, the reflected light reflected by the reflection surface passes through the plurality of hole portions and illuminates the work plate 116 of the work space A.

Consequently, as illustrated in Fig. 6, the gas supplied from the upper front of the work space A by the louver 153 is guided to the back surface side, and also the gas passes through the plurality of hole portions of the top board 119. Consequently, the gas is uniformly supplied into the work space A. Thus, the air current in the work space A is stabilized and contamination is suppressed. Further, as illustrated in Fig. 7, although the intensity of light of the illuminating unit 118 is reduced because the light passes through the plurality of hole portions of the top board 119, it is possible that the illuminating light that has passed through the top board 119 is reflected by the louver 153 to illuminate the work plate 116. This can improve workability of workers.

Note that, the shape of the louver 153 is not limited to a plat plate shape, but may be a shape in which a sectional shape on Y-Z axes plane is a protruded shape to the work space side, that is, the center part between the upper part and the lower part of the louver 153 may have a shape swelling to the illuminating unit 118 side. Thus, it becomes possible to allow the reflected light to be reflected over a wider range, so that a wider range on the work plate 116 can be illuminated. This can further improve workability of workers.

### [Industrial Applicability]

The present disclosure is applicable to an experimental environment device in which a work hand is inserted into a box shaped work space to conduct work.

### [Reference Signs List]

100 isolator system
110 glove box
111 front surface plate
112 front surface opening
113 back surface plate
114 top surface plate
115 bottom surface plate
116 work plate
117 partitioning plate
118 illuminating unit
119 top board
120 centrifuge unit
130 observation unit
140 sterilization unit
150 air conditioning unit
150a intake unit
150b discharge unit
151 inlet
152 particulate trap filter
153 louver
156 outlet
157 particulate trap filter
160 control unit
170 pass box
180 air conditioning unit
190 glove box
200 incubator

## Claims

1. A working chamber comprising:
a box-shaped body case including a work space in an interior thereof and an insertion portion, in a front surface thereof, to which a worker's arm is inserted;
a supply unit configured to supply, into the work space, gas in an exterior of the body case through a supply filter;
a louver that guides gas supplied into the work space by the supply unit to a back surface side of the work space after the gas passes through the supply filter; and
an illuminating means positioned lower than the louver and in front of the louver in the work space, in an upper part of the work space in the body case,
at least one surface of the louver, which faces the illuminating means, being a reflection surface.

2. The working chamber according to claim 1, further comprising:
a discharge filter provided at an upper rear of the body case;
a discharge unit configured to discharge air in the work space to the outside through the discharge filter; and
a partitioning plate that is provided to a back surface side of the body case to form a ventilation passage between a back surface of the body case and the partitioning plate and partition air currents into supply air and discharge air.

3. The working chamber according to claim 1 or 2, wherein the louver includes a part between an upper part and a lower part thereof, which has a shape swelling to the illuminating means side.
